# EUROPÄISCHE PATENTSCHRIFT

(11) **EP 2 199 670 B1**
(45) Veröffentlichungstag und Bekanntmachung des Hinweises auf die Patenterteilung: **31.10.2012**
(21) Anmeldenummer: 08172259.7
(22) Anmeldetag: 19.12.2008
(51) Int. Cl.: F21V 33/00, F21V 21/32, F21V 21/14, F21L 4/00, G02B 6/00, A61B 19/00, F21W 131/20, F21Y 101/02, F21W 131/205

(54) **Mobile Leuchtvorrichtung mit einem Schwanenhals**
Mobile lighting device with a swan neck
Dispositif d'éclairage mobile doté d'un col de cygne

(43) Veröffentlichungstag der Anmeldung: 23.06.2010
(73) Patentinhaber: Asetronics AG, 3018 Bern 18 (CH)
(72) Erfinder: Perrelet, Guido, CH-3098 Köniz (CH)
(74) Vertreter: Rutz & Partner

(56) Entgegenhaltungen:
- EP-A- 0 046 939
- WO-A-03/007047
- GB-A- 971 866
- GB-A- 2 433 110
- US-A- 4 870 964
- US-A- 5 353 786

## Beschreibung

Die Erfindung betrifft eine für die Verwendung im Medizinalbereich vorgesehene mobile Leuchtvorrichtung, die wenigstens eine Leuchtdiode und einen Schwanenhals aufweist.

Eine mobile Leuchtvorrichtung ist aus der EP 1 918 632 A1 bekannt und nachstehend in Figur 2 gezeigt. Diese Leuchtvorrichtung weist eine auf einem Verbindungselement 2' angeordnete Leuchtdiode 82 auf, die über elektrische Leitungen 84 und einen Schalter 83 mit einer Stromversorgungseinheit 81 verbunden ist. Das Verbindungselement 2' weist ein streifenförmiges, flexibles und isolierendes Basissubstrat auf, das mit Metall beschichtet ist, in das die elektrischen Leitungen 84 eingearbeitet sind, die an einem Ende des Verbindungselements 2 zu Anschlüssen 25 der Leuchtdiode 82 und am anderen Ende des Verbindungselements zu Anschlüssen der Stromversorgungseinheit 81 bzw. zum Schalter 83 geführt sind.

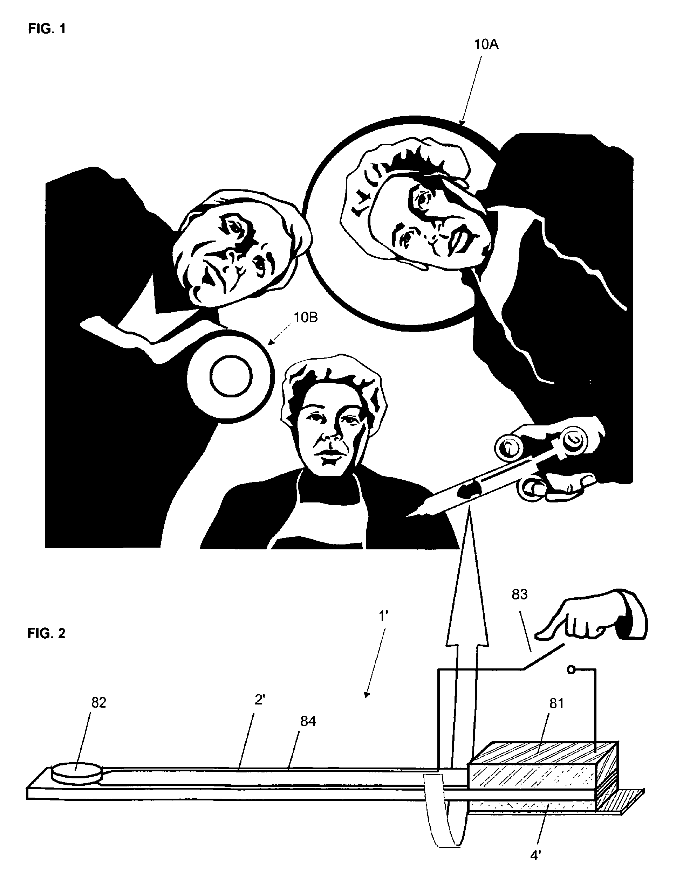

Die Leuchtvorrichtung von Figur 2 ist für die Anwendung im Medizinalbereich vorgesehen, in dem, wie in Figur 1 gezeigt ist, normalerweise fest installierte Operationsleuchten 10A eingesetzt werden, bei denen jedoch störender Schattenwurf auftreten kann. Um dies zu kompensieren wird Hilfspersonal eingesetzt, welches das Operationsfeld mittels einer Handlampe 10B beleuchtet. Dadurch wird der störende Schattenwurf kompensiert, jedoch der Handlungsspielraum des operierenden Arztes eingeschränkt.

Anhand der Leuchtvorrichtung von Figur 2, die anhand einer Verbindungsvorrichtung 4 mit dem Werkzeug des Chirurgen verbunden werden, können diese Probleme vermieden werden. Trotzdem weist auch diese Lösung gewichtige Nachteile auf.

Einerseits ist die Flexibilität des Verbindungselements beschränkt. Es ist darauf zu achten, dass die elektrischen Leitungen während des mehrfachen Biegens des Verbindungselements nicht beschädigt werden. Nachteilig ist ferner die eher klingenartige Ausgestaltung des Verbindungselements, welche den Einsatz im nahen Körperbereich in Frage stellt.

Ferner ist zu beachten, dass die Temperatur der am Ende der Verbindungsvorrichtung angeordneten Leuchtdiode einen zulässigen Wert nicht übersteigt. Weiterhin muss bei dieser Lösung sichergestellt werden, dass das Licht hin zum Operationsfeld abgestrahlt wird und den Operierenden nicht beeinträchtigt.

Ferner ist die Sterilisierung der Leuchtvorrichtung von Figur 2 mit einem relativ hohen Aufwand verbunden, weshalb diese vorzugsweise direkt entsorgt wird.

Weiterhin verursacht die Herstellung der Leuchtvorrichtung von Figur 2 einen relativ hohen Arbeits-, Material- und Kostenaufwand.

Aus der US-A-5 353 786 ist eine gattungsgemäße Leuchtvorrichtung für chirurgische Zwecke bekannt, die einen flexiblen Schlauch mit Öffnungen aufweist zu denen mit einer Lichtquelle verbundene optische Fasern geführt sind.

Aus der WO-A-03/007 047 ist eine Vorrichtung zur Zusatzbeleuchtung eines Objektfeldes unter einem Mikroskop bekannt, bei der die Lichtquelle der Zusatzbeleuchtung am Mikroskop befestigt ist und das Licht über einen innerhalb eines schwanenhalsähnlichen Halters geführten Lichtwellenleiter zum Objektfeld geführt wird.

Aus der US-A-4 870 964 ist ein chirurgisches Gerät für opthalmische Zwecke bekannt, bei dem ein 10-Segment LED Array als Lichtquelle verwendet wird.

Der vorliegenden Erfindung liegt daher die Aufgabe zugrunde, eine kostengünstig herstellbare, und leicht handhabbare mobile Leuchtvorrichtung zu schaffen, welche nicht mit den oben beschriebenen Nachteilen bekannter Lösungen behaftet ist.

Insbesondere soll eine mobile Leuchtvorrichtung geschaffen werden, welche mit einfachen Massnahmen sterilisiert und, ganz oder teilweise, mehrfach verwendet werden kann.

Die mobile Leuchtvorrichtung soll Licht mit genügend hoher Intensität abgeben und den zu bearbeitenden bzw. zu observierenden Gegenstand optimal beleuchten. Dabei soll das Licht auch unter schwierigen Operationsverhältnissen den Arbeitsflächen ohne Schattenbildung gut zuführbar sein.

Die mobile Leuchtvorrichtung soll ferner mit einfachen Massnahmen zuverlässig sterilisierbar sein.

Die Lösung dieser Aufgabe gelingt mit einer mobilen Leuchtvorrichtung gemäss Patentanspruch 1.

Die insbesondere für die Verwendung im Medizinalbereich vorgesehene mobile Leuchtvorrichtung weist einen flexiblen Schwanenhals und eine elektrische Schaltungsanordnung mit wenigstens einer Leuchtdiode auf, die über elektrische Leitungen mit einer Stromversorgungseinheit verbunden oder über einen Schalter verbindbar ist. Möglich ist die Verwendung einzelner Leuchtdioden oder wenigstens eines Leuchtdiodenbündels bzw. LED-Clusters.

Erfindungsgemäss umfasst der Schwanenhals wenigstens einen flexiblen Lichtwellenleiter, der mittels einer Kopplungsvorrichtung lösbar mit einem dicht abgeschlossenen Gehäuse eines Energiemoduls angrenzend an ein daran vorgesehenes Gehäusefenster koppelbar ist, durch das Licht von der wenigstens einen Leuchtdiode, die zusammen mit der gesamten elektrischen Schaltungsanordnung innerhalb des Gehäuses des Energiemoduls eingeschlossen ist, zum Lichtwellenleiter übertragen wird.

Das Gehäusefenster besteht vorzugsweise aus transparentem Glas oder Kunststoff. Das Gehäuse des Energiemoduls kann aus transparentem oder nicht transparentem Glas oder Kunststoff oder weiteren Materialien gefertigt werden.

Das Gehäusefenster ist in das Gehäuse des Energiemoduls eingefügt oder einstückig mit diesem verbunden. Vorzugsweise ist das Gehäuse des Energiemoduls, gegebenenfalls einschliesslich des Gehäusefensters, einteilig in sich geschlossen. Beispielsweise besteht das Gehäuse des Energiemoduls aus transparentem Glas oder transparentem Kunststoff. Das in sich geschlossene Gehäuse des Energiemoduls erlaubt daher keinen Zugriff auf die darin vorgesehene elektrische Schaltung. Gleichzeitig wird verhindert, dass ein Medium in das Energiemodul eindringen oder aus diesem austreten kann. Das Energiemodul kann somit problemlos gereinigt und sterilisiert werden. Damit die Reinigung besonders gut und einfach erfolgen kann, weist das Gehäuse des Energiemoduls vorzugsweise eine glatte Oberfläche ohne Ritzen und Kanten auf. Die elektrische Schaltung, gegebenenfalls vorbehaltlich der Stromversorgungseinheit, wird vorzugsweise in das Energiemodul eingegossen, so dass eine hohe Resistenz gegen mechanische Einwirkungen resultiert.

Der wenigstens eine Lichtwellenleiter besteht vorzugsweise aus Glas oder Kunststoff und weist vorzugsweise einen Durchmesser im Bereich von 1 mm - 12 mm und eine Länge im Bereich von 5 cm - 30 cm auf. Möglich ist auch die Verwendung eines Bündels von Lichtwellenleitern, die anhand eines Klebers miteinander verbunden und/oder in eine Umhüllung eingeschlossen werden.

Vorzugsweise wird ein flexibler und eigenstabiler Lichtwellenleiter verwendet, der in eine bestimmte Richtung gebogen werden kann und in der gewählten Form verharrt. Alternativ kann der Lichtwellenleiter auch in einem flexiblen und eigenstabilen rohrförmigen Körper gehalten sein, der wahlweise ausgerichtet werden kann.

Der Schwanenhals und das Energiemodul können vollständig voneinander getrennt gelagert werden. Typischerweise verfügt der Anwender über wenigstens einen Schwanenhals, an den ein Energiemodul abschliessbar ist. Nach der Entladung des Energiemoduls wird vorzugsweise nur dieses entfernt und ein neues bzw. neu geladenes Energiemodul mit dem Schwanenhals verbunden.

Das Gehäuse des Energiemoduls und/oder der Schwanenhals sind/ist fest oder lösbar mit wenigstens einem Montageelement versehen, mittels dessen das Energiemodul und/oder der Schwanenhals mit einem Arbeitsobjekt, gegebenenfalls einem Operationswerkzeug verbindbar ist. Das Energiemodul und/oder der Schwanenhals können daher je nach Ausgestaltung wahlweise montiert werden. Beispielsweise ist nur der Schwanenhals mit Montageelementen versehen, die mit dem Operationswerkzeug verbunden werden. Das Energiemodul liegt frei und kann mühelos eingesetzt oder ausgetauscht werden.

Das lösbar oder einstückig mit dem Gehäuse des Energiemoduls bzw. dem Schwanenhals verbundene wenigstens eine Montageelement ist z.B. eine Klammer, ein Clip, oder eine Klemme aus Metall und/oder Kunststoff. Vorzugsweise kann der Anwender die passenden Montageelemente aus einem Sortiment frei wählen.

Die Kopplungsvorrichtung weist ein mit dem Schwanenhals verbindbares Aufnahmeteil auf. Dieses Aufnahmeteil kann einstückig am Gehäuse des Energiemoduls befestigt bzw. daran angeformt oder mittels eines Befestigungselements mit dem Gehäuse des Energiemoduls verbindbar sein. Sofern das Aufnahmeteil am Gehäuse des Energiemoduls angeformt ist, resultiert ein minimaler Aufwand. Weitere Montagemittel sind in diesem Fall nicht erforderlich. Vorzugsweise weist das angeformte Aufnahmeteil federelastische Finger auf, mittels derer der Schwanenhals gehalten wird.

Das gegebenenfalls vorgesehene Befestigungselement der Kopplungsvorrichtung ist vorzugsweise mit einem Halteelement koppelbar, das vorzugsweise einstückig am Gehäuse des Energiemoduls angeformt ist.

Alternativ kann das Befestigungselement ein der Aufnahme des Energiemoduls dienendes U-Profil aufweisen, dessen Seitenwände vorzugsweise als Klammern ausgestaltet sind, welche das Energiemodul festhalten.

Weiterhin kann das Befestigungselement aus Metall gefertigt sein und mit einem im Energiemodul vorgesehenen Kopplungsmagneten zusammenwirken. Durch den beispielsweise in das Gehäuse des Energiemoduls eingegossenen Kopplungsmagneten kann das Befestigungselement gegen das Gehäuse vorzugsweise in eine darin vorgesehene Einsenkung gezogen werden. Innerhalb der Einsenkung ist das Befestigungselement seitlich nicht verschiebbar, so dass ein seitliches Abscheren des Befestigungselements ausgeschlossen ist. Möglich ist jedoch auch die Verwendung eines magnetischen Befestigungselements, welches mit einem Metallkörper zusammenwirkt, der im Energiemodul vorgesehen ist.

In einer vorzugsweisen Ausgestaltung ist der Kopplungsmagnet ringförmig ausgestaltet und angrenzend an das Gehäusefenster derart angeordnet, dass das von der wenigstens einen Leuchtdiode abgegebene Licht durch den Kopplungsmagneten hindurch treten kann. Auf diese Weise wird eine optimale Wirkung des Kopplungsmagneten sowie eine gute Ankopplung der Leuchtdiode an das Gehäusefenster bzw. an den Lichtwellenleiter erzielt. Dazu kann die Leuchtdiode auch axial in den gleichzeitig als Halterung dienenden Kopplungsmagneten hinein ragen.

In einer weiteren vorzugsweisen Ausgestaltung ist ein Montagemagnet am Befestigungselement vorgesehen, der Kraft auf ein im Energiemodul vorgesehenes magnetisches Element ausübt. In dieser Ausgestaltung kann das Energiemodul ohne Kraftaufwand in die Kopplungsvorrichtung eingesetzt werden. Auch bei dieser Ausgestaltung ist die Verwendung eines magnetischen Befestigungselements möglich, welches mit einem Metallkörper zusammenwirkt, der im Energiemodul vorgesehen ist.

Die Verwendung des Kopplungsmagneten oder des Montagemagneten erlaubt ferner die automatische Betätigung der Leuchtvorrichtung. Dabei wird vorgesehen, dass der mit der Leuchtdiode und der Stromversorgungseinheit verbundene Schalter automatisch betätigt wird, wenn das Energiemodul mit dem Schwanenhals verbunden wird.

Wesentlich beim Einsatz von Magneten ist, dass zwei zusammenwirkende magnetische Elemente vorgesehen sind, von denen wenigstens eines hartmagnetisch ist. Dabei wird das hartmagnetische Element, der Kopplungsmagnet oder der Montagemagnet, vorzugsweise innerhalb des Energiemoduls vorgesehen. Wie dies nachstehen jedoch gezeigt ist, kann der Montagemagnet auch Teil der externen Kopplungsvorrichtung sein.

Der Kopplungsmagnet und/oder der Montagemagnet bestehen vorzugsweise aus hartmagnetischen Materialien, die aus seltenen Erden gefertigt sind. Hartmagnetische Werkstoffe sind beispielsweise Kobalt-Samarium (SmCo5, Sm2Co17, Sm (Co, Cu, Fe, Zr) und Neodym-Eisen-Bor (NdFeB).

In einer weiteren vorzugsweisen Ausgestaltung weist das Gehäuse des Energiemoduls eine elastische Zone auf, welche die manuelle Betätigung des Schalters erlaubt. Die elektrische Schaltung im Energiemodul kann dadurch gesteuert werden, ohne dass Bedienelemente aus dem Gehäuse des Energiemoduls herausgeführt werden müssen.

In einer weiteren vorzugsweisen Ausgestaltung weist das Gehäuse des Energiemoduls eine Sollbruchstelle auf, die derart ausgestaltet ist, dass deren Auftrennung wenigstens den Zugang zur Stromversorgungseinheit öffnet. Dadurch ist es möglich, die verschiedenen Elemente eines erschöpften oder defekten Energiemoduls zu separieren.

In einer weiteren vorzugsweisen Ausgestaltung ist die Stromversorgungseinheit vorzugsweise über eine Diode mit einer Spule verbunden, die mit der Spule eines Ladegeräts vorzugsweise kontaktlos koppelbar ist. Das Energiemodul kann daher mehrmals regeneriert bzw. neu geladen werden. Grundsätzlich kann als Stromversorgungseinheit eine Batterie oder ein Akkumulator verwendet werden.

Die Erfindung wird nachstehend anhand von Zeichnungen näher erläutert. Dabei zeigt:
- Fig. 1: in einem Operationssaal verwendete Mittel 10A, 10B zur Beleuchtung eines Arbeitsfeldes;
- Fig. 2: die eingangs beschriebene bekannte Leuchtvorrichtung 1';
- Fig. 3a: eine erfindungsgemässe Leuchtvorrichtung 1 mit einem Schwanenhals 2, der mittels einer Kopplungsvorrichtung 5 lösbar mit einem Energiemodul 3 gekoppelt ist;
- Fig. 3b: die Leuchtvorrichtung 1 mit dem vom Energiemodul 3 gelösten Schwanenhals 2;
- Fig. 4a: ein auf den Schwanenhals 2 aufgesetztes Montageelemente 44;
- Fig. 4b: das auf den Schwanenhals 2 aufgesetzte und mit einem Arbeitsobjekt 7 verbundene Montageelement 44 von Figur 4a;
- Fig. 5: das Energiemodul 3 von Figur 3a, dessen Gehäuse 30 einstückig mit einem Montageelement 34 verbunden ist;
- Fig.6: eine erfindungsgemässe Leuchtvorrichtung 1 mit einem Energiemodul 3, welches in das U-Profilförmig ausgestaltete und mit einem Montageelement 54 versehene Befestigungselement 52 der Kopplungsvorrichtung 5 eingesetzt wird;
- Fig. 7: eine erfindungsgemässe Leuchtvorrichtung 1 mit einem weiteren vorzugsweise ausgestalteten Energiemodul 3, welches beim Einsetzen in das Befestigungselement 52 der Kopplungsvorrichtung 5 mittels eines Montagemagneten 58 gehalten und automatisch aktiviert wird;
- Fig. 8a: in schematischer Darstellung ein erfindungsgemässes Energiemodul 3, in dem ein ringförmiger Magnet 350 vorgesehen ist, mittels dessen das Befestigungselement 52 der Kopplungsvorrichtung 5 gehalten wird;
- Fig. 8b: das Energiemodul 3 von Figur 8a mit dem durch den Magneten 350 in einer Einsenkung 305 des Gehäuses 30 gehaltenen Befestigungselement 52 und mit einem Ladegerät 9; und
- Fig. 9: ein Energiemodul 3 mit einem Gehäuse 30, an dem das Aufnahmeelement 35 der Kopplungsvorrichtung 5 einstückig angeformt ist.

Figur 1 zeigt die eingangs erwähnten Mittel 10A, 10B zur Beleuchtung eines Arbeitsfeldes in einem Operationssaal.
Figur 2 zeigt die eingangs beschriebene und aus der EP 1 918 632 A1 bekannte Leuchtvorrichtung 1'.

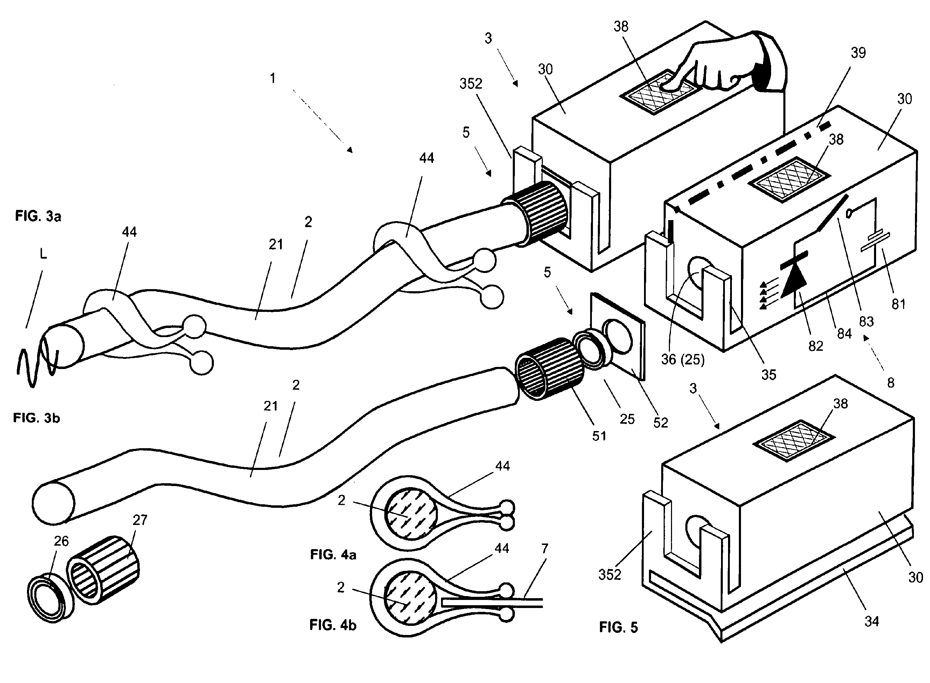

Figur 3a zeigt eine erfindungsgemässe Leuchtvorrichtung 1 mit einem Schwanenhals 2, der mittels einer Kopplungsvorrichtung 5 lösbar mit einem Energiemodul 3 gekoppelt ist. Figur 3b zeigt die Leuchtvorrichtung 1 mit dem vom Energiemodul 3 gelösten Schwanenhals 2.

Der Schwanenhals 2 umfasst im Wesentlichen einen Lichtwellenleiter 21, durch den vom Energiemodul 3 abgegebenes Licht L hin zum Operationsfeld übertragen wird. Der Schwanenhals 2 bzw. der Lichtwellenleiter 21 ist vorzugsweise derart beschaffen, dass er flexibel in eine bestimmte Form gebracht werden kann und eigenstabil in dieser Form verharrt. Mittels der gezeigten Montageelemente 44, vorzugsweise einfachen Klemmen, wie sie auch in den Figuren 4a und 4b gezeigt sind, kann der Schwanenhals 2 bzw. der vorzugsweise einstückige Lichtwellenleiter 21 mit einem Arbeitsobjekt, beispielsweise einem Operationswerkzeug 7 verbunden werden. Dabei sind mehrere Montageelemente 44 einsetzbar, die vorzugsweise fest, gegebenenfalls lösbar mit dem Schwanenhals 2 verbunden sind.

Sofern der Schwanenhals 2 bzw. der Lichtwellenleiter 21 nicht eigenstabil ist, so kann dieser anhand der Montageelemente 44 ebenfalls in einer gewünschten Form und Lage fixiert werden. Der Lichtwellenleiter 21 kann ein einzelnes flexibles Glasrohr oder Kunststoffrohr sein oder kann aus mehreren Lichtwellenleitern mit kleinerem Durchmesser zusammengesetzt sein. Der Gesamtquerschnitt des Lichtwellenleiters bzw. der Lichtwellenleiter 21 weist vorzugsweise die Form eines Kreises auf, kann jedoch jede beliebige weitere Form, z.B. eine Rechteckform aufweisen. Durch die Wahl der Form des Lichtwellenleiters kann dessen Biegeverhalten wahlweise beeinflusst werden. Mit rundem Querschnitt weist der Lichtwellenleiter keine bevorzugte Biegerichtung auf. Mit rechteckigem Querschnitt resultieren bevorzugte Biegeachsen, die senkrecht zueinander stehen.

In den Figuren 3a und 3b ist weiter gezeigt, dass der Lichtwellenleiter 21 in ein hohlzylindrisches oder hülsenförmiges Aufnahmeteil 51 einer aus Kunststoff oder Metall gefertigten Kopplungsvorrichtung 5 einführbar ist. Das Aufnahmeteil 51 ist fest mit einem rechteckigen Befestigungselement 52 verbunden, welches eine an das Aufnahmeteil 51 angepasste Durchtrittsöffnung aufweist. Das Befestigungselement 52 ist in Figur 3a in ein am Gehäuse 30 des Energiemoduls 3 angeformtes, gabelförmiges Halteelement 352 eingehängt, welches das Aufnahmeteil 51 umgreift und das Befestigungselement 52 vorzugsweise festklemmt. Dazu sind das Befestigungselement 52 und das Halteelement 352 vorzugsweise entsprechend gebogen oder mit elastischen Klemmelementen versehen. Das Aufnahmeteil 51 und das Befestigungselement 52 sind dabei derart im Halteelement 352 fixiert, dass die Längsachse des Lichtwellenleiters 21 durch den Mittelpunkt eines Gehäusefensters 36 verläuft, das im Gehäuse 30 des Energiemoduls 3 vorgesehen ist.

Das Gehäusefenster 36 kann in das Gehäuse 30 des Energiemoduls 3 eingesetzt oder einstückig darin vorgesehen sein. Beispielsweise wird das Gehäuse 30 des Energiemoduls 3 aus transparentem Kunststoff oder Glas gefertigt, so dass das Gehäusefenster 36 nicht speziell vorgesehen werden muss.

Innerhalb des Energiemoduls 3 ist angrenzend an das Gehäusefenster 36 eine Leuchtdiode 82 vorgesehen, die in einer prinzipiellen Ausgestaltung über einen Schalter 83 und elektrische Leitungen 84 mit einer Stromversorgungseinheit 81 verbunden ist. In der gezeigten vorzugsweisen Ausgestaltung ist der Schalter 83 derart angeordnet, dass er durch ein bewegbares Teil 38 des Gehäuses 3 betätigt werden kann. Vorzugsweise ist das bewegbare Teil 38 durch Sicken, die z.B. mit reduzierter Dicke wellenförmig verlaufen, eingeschlossen. Die Sicken erlauben, analog zu einer Lautsprechermembran, die Auslenkung des bewegbaren Teils 38 und somit die Betätigung des Schalters 83.

Nach der Betätigung des vorzugsweise mit einem Federelement versehenen Schalters 83 wird von der Leuchtdiode 82, gegebenenfalls einem Bündel von Leuchtdioden, Licht durch das Gehäusefenster 36 in der Lichtwellenleiter 21 eingekoppelt und durch diesen zum Arbeitsfeld übertragen.

Die Figuren 4a und 4b zeigen ein auf den Schwanenhals 2 aufgesetztes Montageelemente 44. Figur 4b zeigt den anhand des Montageelements 44 mit einem Arbeitsobjekt 7 verbunden Schwanenhals 2.

Figur 5 zeigt ein vorzugsweise ausgestaltetes Energiemodul 3, dessen Gehäuse 30 einstückig mit einem Montageelement 34 verbunden ist. Zwischen dem als Klammer dienenden Montageelement 34 und dem Gehäuse 3 kann ein Arbeitsobjekt 7 gehalten werden. Das Energiemodul 3 kann daher ohne weitere Hilfsmittel einfach montiert werden.

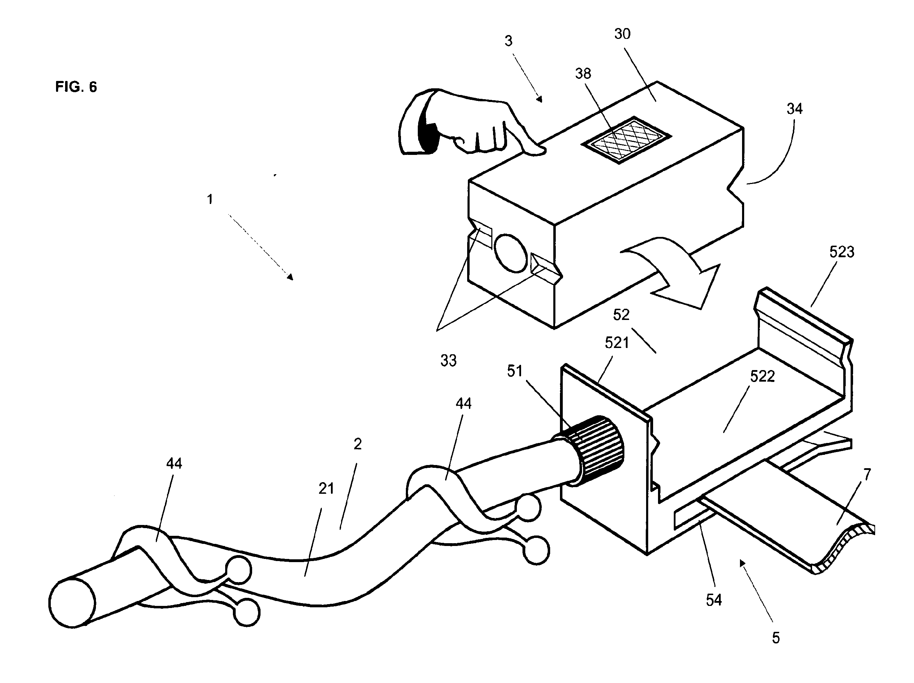

Figur 6 zeigt eine erfindungsgemässe Leuchtvorrichtung 1 mit einem vorzugsweise ausgestalteten Energiemodul 3, welches in vorzugsweise ausgestaltetes Befestigungselement 52 der Kopplungsvorrichtung 5 eingesetzt wird. Das mit einem Montageelement 54 versehene Befestigungselement 52 weist ein U-Profil mit zwei vorzugsweise federelastischen Seitenwänden 521, 523 auf, welche in Ausnehmungen 33, 34 im Gehäuse 30 des Energiemoduls 3 eingreifen und dieses halten, gegebenenfalls gegen das Mittelteil 522 des Befestigungselements 52 drücken. Das Befestigungselement 52 bildet daher eine Art Clip- oder Schnappvorrichtung, in die das Energiemodul 3 eingesetzt werden kann. Das Montageteil 54 erlaubt die Montage des Befestigungselements 52 an einem Arbeitsobjekt 7. Wie bereits in Figur 3a gezeigt ist, kann auch der Lichtwellenleiter 21 mittels Montageelementen 44 mit dem Arbeitsobjekt 7 verbunden werden.

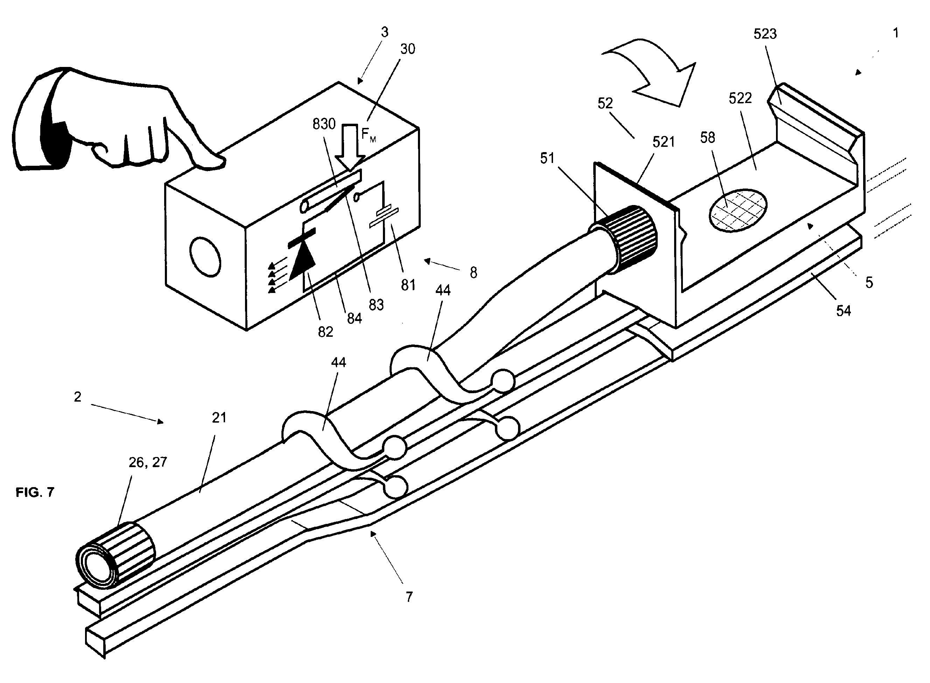

Wie dies auch in Figur 7 gezeigt ist, können der Schwanenhals 2 und die Kopplungsvorrichtung 5 bei den Vorbereitungsarbeiten zu einem chirurgischen Eingriff auf einem Operationswerkzeug 7 vormontiert werden. In Figur 7 kann das mit dem Befestigungselement 52 verbundene Montageelement 54 seitlich am Operationswerkzeug 7 festgeklemmt werden.

Ein passendes Energiemodul 3, beispielsweise das Energiemodul 3 von Figur 6 oder das vorzugsweise ausgestaltete Energiemodul 3 von Figur 7, können bei Arbeitsbeginn in das Befestigungselement 52 eingesetzt werden. Nachdem das Energiemodul 3 erschöpft ist, kann es durch ein neues ersetzt werden.

Damit das Energiemodul 3 leichter montiert werden kann, ist in der Mittelplatte 522 des Befestigungselements 52 ein hartmagnetischer Montagemagnet 58 eingesetzt, durch den das Energiemodul 3, welches mit einem weich- oder hartmagnetischen Gegenelement 830 versehen ist, in das Befestigungselement 52 hineingezogen wird. Das Gegenelement 830 ist beispielsweise drehbar gelagert und durch eine Feder gestützt. Durch Einwirkung der Kraft des Montagemagneten 58 wird die Feder zusammengerückt und der Schaltkontakt 83 geschlossen. Das Energiemodul 3 kann daher mit Hilfe des Montagemagneten 58 einfach montiert und automatisch aktiviert werden.

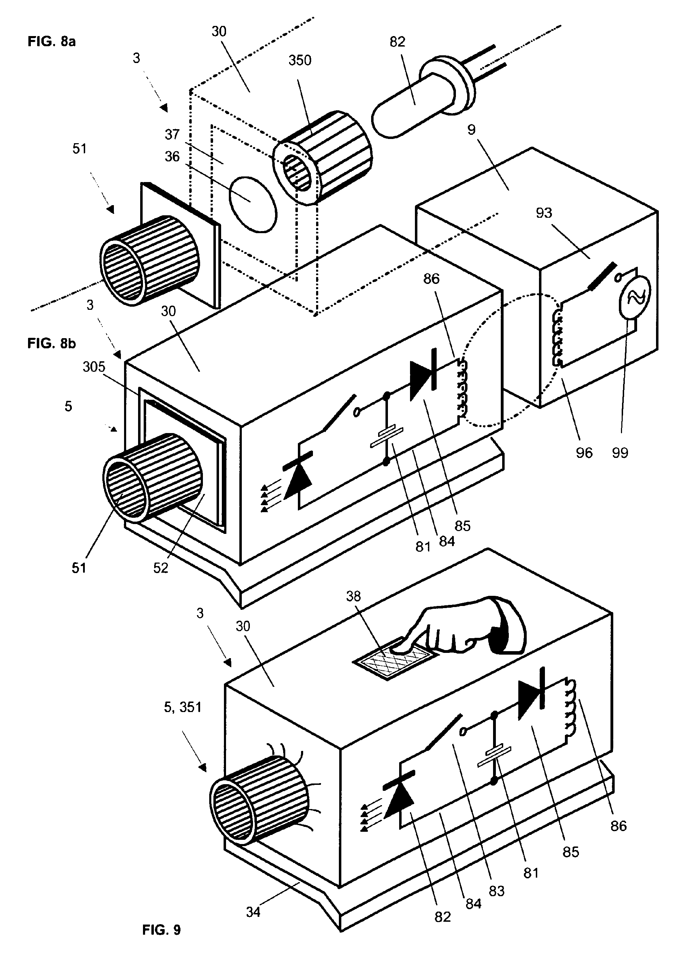

Figur 8a zeigt in schematischer Darstellung ein vorzugsweise ausgestaltetes Energiemodul 3, in dem ein ringförmiger Kopplungsmagnet 350 vorgesehen ist, mittels dessen das Befestigungselement 52 der Kopplungsvorrichtung 5 gehalten wird. Der Kopplungsmagnet 350 ist beispielsweise neben dem Gehäusefenster 36 im Gehäuse 30 eingegossen. Die Leuchtdiode 82 ist vorzugsweise in den hohlzylindrischen Körper des Kopplungsmagneten 350 eingeführt, so dass wenig Platz beansprucht und eine optimale optische Kopplung durch das Gehäusefenster 36 erzielt wird. Vorzugsweise weist das Gehäuse 30 im Bereich des Gehäusefensters 36 eine Einsenkung 37 auf, die der Aufnahme des Befestigungselements 52 dient. Das Befestigungselement 52 wird durch den Kopplungsmagneten 350 in die Einsenkung 37 hineingezogen und dort durch die Einsenkung 34 seitlich formschlüssig und durch den Kopplungsmagneten 350 kraftschlüssig gehalten. Möglich ist auch die Verwendung eines hartmagnetischen Befestigungselements, welches mit einem weichmagnetischen, gegebenenfalls zur Betätigung des Schalters 83 dienenden Gegenelement 830 zusammenwirkt.

Figur 8b zeigt das Energiemodul 3 von Figur 8a mit dem durch den Magneten 350 in einer Einsenkung 305 des Gehäuses 30 gehaltenen Befestigungselement 52. Ferner ist ein Ladegerät 9 mit einer Wechselstromquelle 99, einem Schalter 93 und einer ersten Ladespule 96 gezeigt, die induktiv an eine im Energiemodul 3 vorgesehene zweite Ladespule 86 angekoppelt ist. Die in der zweiten Ladespule 86 induzierte Wechselspannung wird durch eine Diode 85 gleichgerichtet und der Stromversorgungseinheit 81 zugeführt. Die zweite Ladespule 86 wird vorzugsweise derart angeordnet, dass ein maximaler Spulenquerschnitt resultiert. Eine besonders gute Zusammenwirkung der Ladespulen 86, 96 wird erzielt, wenn diese aneinander liegen oder ineinander verschoben werden. Beispielsweise wird vorgesehen, dass das Energiemodul 3 in eine Öffnung in der Ladestation 9 hinein verschoben werden kann.

Ein erfindungsgemässes Energiemodul 3 kann nach Gebrauch gereinigt, sterilisiert und kontaktlos wieder geladen werden. Ein Eingriff in das Energiemodul 3 hinein oder eine Stromzufuhr über Leitungen ist daher zu keinem Zeitpunkt erforderlich. Lediglich zur Entsorgung des Energiemoduls 3 ist vorzugsweise eine Sollbruchstelle 39 vorgesehen (siehe Figur 3a), welche es erlaubt, das Gehäuse 30 zu öffnen und die darin vorgesehenen Materialien geordnet zu entsorgen oder zu rezyklieren.

Figur 9 zeigt ein Energiemodul 3 mit einem Gehäuse 30, an dem das Aufnahmeelement 35 der Kopplungsvorrichtung 5 einstückig angeformt ist. Durch diese Massnahme gelingt es, die Kopplungsvorrichtung 5 weiter zu vereinfachen bzw. auf das am Gehäuse vorgesehene Aufnahmeelement 35 zu reduzieren.

Die erfindungsgemässe Leuchtvorrichtung wurde in verschiedenen prinzipiellen Ausgestaltungen gezeigt, die in fachmännischer Weise weitergebildet werden können. Insbesondere wird der Fachmann ästhetisch und funktional sinnvolle Formen für das Gehäuse 30 des Energiemoduls 3 schaffen. Dabei werden vorzugsweise abgerundete Formen ohne Ecken und Kanten verwendet.

Möglich ist ferner die Verwendung von Prozessoren und Netzwerkmodulen, welche es erlauben, die Leuchtdioden 82 gezielt anzusteuern und wahlweise ein- oder auszuschalten. Möglich ist auch die Steuerung der Lichtart und Intensität, indem beispielsweise Leuchtdioden mit unterschiedlichen Farben zu oder abgeschaltet werden. Weiterhin kann das Gehäusefenster auch mit einem Farbfilter versehen sein.

## Patentansprüche

1. Mobile Leuchtvorrichtung (1), insbesondere für die Verwendung im Medizinalbereich, mit einem flexiblen Schwanenhals (2), der wenigstens einen flexiblen Lichtwellenleiter (21) umfasst, und mit einer elektrischen Schaltungsanordnung (8) umfassend wenigstens eine Leuchtdiode (82), die über elektrische Leitungen (84) mit einer Stromversorgungseinheit (81) verbunden oder über einen Schalter (83) verbindbar ist, wobei die Leuchtdiode (82) zusammen mit der gesamten elektrischen Schaltungsanordnung (8) innerhalb des Gehäuses (3) eines Energiemoduls (3) eingeschlossen ist, **dadurch gekennzeichnet, dass** der Lichtwellenleiter (21) mittels einer Kopplungsvorrichtung (5, 352; 35; 350) lösbar mit dem dicht abgeschlossenen Gehäuse (30) des Energiemoduls (3) angrenzend an ein daran vorgesehenes Gehäusefenster (36) koppelbar ist, das aus transparentem Glas oder Kunststoff besteht und durch das hindurch Licht von der wenigstens einen Leuchtdiode (82) zum Lichtwellenleiter (21) übertragbar ist.

2. Mobile Leuchtvorrichtung (1) nach Anspruch 1, **dadurch gekennzeichnet, dass** das Gehäuse (30) des Energiemoduls (3) aus transparentem oder nicht transparentem Glas oder Kunststoff besteht.

3. Mobile Leuchtvorrichtung (1) nach Anspruch 1 oder 2, **dadurch gekennzeichnet, dass** das Gehäusefenster (36) in das Gehäuse (30) des Energiemoduls (3) eingefügt oder einstückig mit diesem verbunden ist und/oder dass das Gehäuse (30) des Energiemoduls (3) einteilig in sich geschlossen ist.

4. Mobile Leuchtvorrichtung (1) nach Anspruch 1, 2 oder 3, **dadurch gekennzeichnet, dass** der wenigstens eine aus Glas oder Kunststoff bestehende Lichtwellenleiter (21) einen Durchmesser im Bereich von 1 mm - 12 mm und eine Länge im Bereich von 5 cm - 30 cm aufweist.

5. Mobile Leuchtvorrichtung (1) nach einem der Ansprüche 1 - 4, **dadurch gekennzeichnet, dass** das Gehäuse (30) des Energiemoduls (3) und/oder der Schwanenhals (2) fest oder lösbar mit wenigstens einem Montageelement (34; 44; 54) versehen ist, mittels dessen das Energiemodul (3) und/oder der Schwanenhals (2) mit einem Arbeitsobjekt (7) verbindbar ist.

6. Mobile Leuchtvorrichtung (1) nach 5, **dadurch gekennzeichnet, dass** das lösbar oder einstückig mit dem Gehäuse (30) des Energiemoduls (3) bzw. dem Schwanenhals (2) verbundene wenigstens eine Montageelement (34; 44; 54) eine Klammer, ein Clip, oder eine Klemme aus Metall und/oder Kunststoff ist.

7. Mobile Leuchtvorrichtung (1) nach Anspruch 4, 5 oder 6, **dadurch gekennzeichnet, dass** die Kopplungsvorrichtung (5, 352; 35; 350) ein mit dem Schwanenhals (2) verbindbares Aufnahmeteil (35; 51) aufweist, das vorzugsweise einstückig am Gehäuse (30) des Energiemoduls (3) befestigt bzw. mit diesem verbunden ist oder mittels eines Befestigungselements (52) mit dem Gehäuse (30) des Energiemoduls (3) verbindbar ist.

8. Mobile Leuchtvorrichtung (1) nach Anspruch 7, **dadurch gekennzeichnet, dass**
a) dass das Befestigungselement (52) mit einem vorzugsweise einstückig am Gehäuse (30) des Energiemoduls (3) angeformten Halteelement (352) koppelbar ist; oder
b) dass das Befestigungselement (52) ein der Aufnahme des Energiemoduls (3) dienendes U-Profil (521, 522, 523) bildet, dessen Seitenwände (521, 523) vorzugsweise als Klammern ausgestaltet sind, welche das Energiemodul (3) festhalten; oder
c) dass das metallene Befestigungselement (52) mittels eines Kopplungsmagneten (350) vorzugsweise in einer im Gehäuse (30) des Energiemoduls (3) vorgesehenen Einsenkung (37) gehalten ist; oder
d) dass das metallene Befestigungselement (52) einen Montagemagneten (58) aufweist, welcher das mit einem weich- oder hartmagnetischen Gegenelement (830) versehene Energiemodul (3) innerhalb des Befestigungselements (52) hält.

9. Mobile Leuchtvorrichtung (1) nach Anspruch 8, **dadurch gekennzeichnet, dass** der Magnet (350) ringförmig ausgestaltet und angrenzend an das Gehäusefenster (36) derart angeordnet ist, dass das von der wenigstens einen Leuchtdiode (82) abgegebene Licht durch den Magneten (350) hindurch treten kann und/oder dass die Leuchtdiode (82) axial in den Magneten (350) hinein ragt.

10. Mobile Leuchtvorrichtung (1) nach einem der Ansprüche 1-9, **dadurch gekennzeichnet, dass** das Gehäuse (30) des Energiemoduls (3) eine elastische Zone (38) aufweist, welche die manuelle Betätigung des Schalters (83) erlaubt.

11. Mobile Leuchtvorrichtung (1) nach einem der Ansprüche 1-10, **dadurch gekennzeichnet, dass** das Gehäuse (30) des Energiemoduls (3) eine Sollbruchstelle (39) aufweist, die derart ausgestaltet ist, dass deren Auftrennung wenigstens den Zugang zur Stromversorgungseinheit (81), einer Batterie oder einem Akkumulator, öffnet.

12. Mobile Leuchtvorrichtung (1) nach einem der Ansprüche 1-10, **dadurch gekennzeichnet, dass** die Stromversorgungseinheit (81) vorzugsweise über eine Diode (85) mit einer Spule (86) verbunden ist, die mit der Spule (96) eines Ladegeräts (9) vorzugsweise kontaktlos koppelbar ist.

13. Mobile Leuchtvorrichtung (1) nach einem der Ansprüche 1 bis 12, **dadurch gekennzeichnet, dass** der Schalter (83) derart ausgestaltet und positioniert ist und/oder mit einem beweglich gelagerten Gegenelement (830) zusammenwirkt, dass er durch Einwirkung des Montagemagneten (58) oder des Kopplungsmagneten (350) schliessbar ist.

14. Mobile Leuchtvorrichtung (1) nach einem der Ansprüche 1-13, **dadurch gekennzeichnet, dass** der wenigstens eine Lichtwellenleiter (21), gegebenenfalls einen Bündel von Lichtwellenleitern (21) flexibel und eigenstabil oder in einem flexiblen und eigenstabilen rohrförmigen Körper gehalten ist.

15. Mobile Leuchtvorrichtung (1) nach einem der Ansprüche 1-14, **dadurch gekennzeichnet, dass** das Gehäuse (30) des Energiemoduls (3) eine glatte Oberfläche aufweist.

## Claims

1. Mobile lighting device (1), particularly for use in the medical field, with a flexible swan neck (2), with at least one flexible light conductor (21), and with an electrical circuit arrangement (8) comprising at least one light emitting diode (82) that via electrical lines (84) is connected or connectable via a switch (83) with a power supply unit (81), wherein the light emitting diode (82) together with the complete electrical circuit arrangement (8) is enclosed within the housing (3) of an energy module (3), **characterized in that** the light conductor (21) is detachably connectable by means of a coupling device (5, 352; 35; 350) with the tightly sealed housing (30) of the energy module (3), adjacent to a housing window (36), which is provided on the housing (3) and consists of transparent glass or plastic and through which light of the at least one light emitting diode (82) is transferable to the light conductor (21).

2. Mobile lighting device (1) according to claim 1, **characterized in that** the housing (30) of the energy module (3) consists of transparent or non-transparent glass or plastic.

3. Mobile lighting device (1) according to claim 1 or 2, **characterized in that** the housing window (36) is inserted into the housing (30) of the energy module (3) or is incorporated in one piece in the housing (30) of the energy module (3) and/or that the housing (30) of the energy module (3) is closed in itself in one part.

4. Mobile lighting device (1) according to claim 1, 2 or 3, **characterized in that** the at least one light conductor (21), which consists glass or plastic, has a diameter in the range of 1 mm - 12 mm and a length in the range of 5 cm - 30 cm.

5. Mobile lighting device (1) according to one of the claims 1 - 4, **characterized in that** the housing (30) of the energy module (3) and/or the swan neck (2) is provided firmly or detachably with at least one mounting element (34; 44; 54), with which the energy module (3) and/or the swan neck (2) is connectable with a work item (7).

6. Mobile lighting device (1) according to claim 5, **characterized in that** the at least one mounting element (34; 44; 54), which is detachably or in one piece connected to the housing (30) of the energy module (3) or the swan neck (2) respectively, is at least one bracket, a clip, or a clamp made from metal and/or plastic.

7. Mobile lighting device (1) according to claim 4, 5 or 6, **characterized in that** the coupling device (5, 352; 35; 350) is provided with a receiving part (35; 51), which is connectable to the swan neck (2) and which preferably is mounted in one piece to the housing (30) of the energy module (3) or is connectable to the housing (30) of the energy module (3) by means of a the mounting element (52) respectively.

8. Mobile lighting device (1) according to claim 7, **characterized in that**
a) that the mounting element (52) is connectable with holding element (352) that is preferably formed in one piece on the housing (30) of the energy module (3); or
b) that the mounting element (52) forms a U-profile (521, 522, 523) serving for accommodating the energy module (3), said U-profile (521, 522, 523) comprising side walls (521, 523) preferable in the embodiment of brackets, which hold the energy module (3); or
c) that the metal mounting element (52) is held by means of a coupling magnet (350) preferably in a recess (37) provided in the housing (30) of the energy module (3); or
d) that the metal mounting element (52) comprises a mounting magnet (58), which holds the energy module (3), which is provided with a soft- or hard magnetic counter element (830), within the mounting element (52).

9. Mobile lighting device (1) according to claim 8, **characterized in that** the magnet (350) has an annular form and is located adjacent to the housing window (36) in such a way, that the light provided by the at least one light emitting diode (82) can pass through the magnet (350) and/or that the light emitting diode (82) axially projects into the magnet (350).

10. Mobile lighting device (1) according to one of the claims 1-9, **characterized in that** the housing (30) of the energy module (3) comprises an elastic zone (38), which allows manually actuating the switch (83).

11. Mobile lighting device (1) according to one of the claims 1-10, **characterized in that** the housing (30) of the energy module (3) comprises a predetermined breaking point (39) designed in such a way, that its breakage at least opens access to the power supply unit (81), a battery or an accumulator.

12. Mobile lighting device (1) according to one of the claims 1-10, **characterized in that** the power supply unit (81) is connected, preferably via a diode (85), with a coil (86), which can be coupled, preferably contactless, with a coil (96) of a charging unit (9).

13. Mobile lighting device (1) according to one of the claims 1 - 12, **characterized in that** the switch (83) is designed and positioned in such a way and/or interacts with a movable suspended counter element (830), that it is closable under impact of the mounting magnet (58) or the coupling magnet (350).

14. Mobile lighting device (1) according to one of the claims 1-13, **characterized in that** the at least one light conductor (21), if appropriate a bundle of light conductors (21), is held flexible and self-stable or within a flexible and self-stable tubular body.

15. Mobile lighting device (1) according to one of the claims 1-14, **characterized in that** the housing (30) of the energy module (3) exhibits a smooth surface.

## Revendications

1. Dispositif d'éclairage mobile (1), en particulier pour l'utilisation dans le secteur médical, avec un col de cygne flexible (2) qui comprend au moins un conducteur d'ondes lumineuses flexible (21), et avec un dispositif de circuit électrique (8) comprenant au moins une diode lumineuse (82) qui est reliée par des lignes électriques (84) à une unité d'alimentation de courant (81) ou par un interrupteur (83), la diode lumineuse (82) étant encastrée ensemble avec l'ensemble du dispositif de circuit électrique (8) à l'intérieur du coffret (3) d'un module d'énergie (3), **caractérisé en ce que** le conducteur d'ondes lumineuses (21) peut être accouplé au moyen d'un dispositif de couplage (5, 352 ; 35 ; 350) de manière amovible au coffret étanche (30) du module d'énergie (3) à proximité d'une fenêtre de coffret (36) prévue à cet effet, qui se compose de verre transparent ou de matière plastique transparent et par laquelle la lumière d'au moins une diode lumineuse (82) peut être transmise au conducteur d'ondes lumineuses (21).

2. Dispositif lumineux mobile (1) selon la revendication 1, **caractérisé en ce que** le coffret (30) du module d'énergie (3) se compose de verre ou matière plastique transparent ou non-transparent.

3. Dispositif lumineux mobile (1) selon la revendication 1 ou 2, **caractérisé en ce que** la fenêtre de coffret (36) est introduite dans le coffret (30) du module d'énergie (3) ou reliée à ce dernier d'un seul morceau et/ou **en ce que** le coffret (30) du module d'énergie (3) est fermé sur lui-même d'une seule pièce.

4. Dispositif lumineux mobile (1) selon la revendication 1, 2 ou 3, **caractérisé en ce qu'**au moins un conducteur d'ondes lumineuses (21) se composant de verre ou de matière plastique présente un diamètre compris entre 1 mm et 12 mm et une longueur de 5 cm à 30 cm.

5. Dispositif lumineux mobile (1) selon l'une des revendications 1 à 4, **caractérisé en ce que** le coffret (30) du module d'énergie (3) et/ou le col de cygne (2) est muni de manière fixe ou amovible d'au moins un élément de montage (34 ; 44 ; 54) au moyen duquel le module d'énergie (3) et/ou le col de cygne (2) peut être connecté à un objet de travail (7).

6. Dispositif lumineux mobile (1) selon la revendication 5, **caractérisé en ce qu'**au moins un élément de montage (34 ; 44 ; 54) relié de manière amovible ou d'une seule pièce au coffret (30) du module d'énergie (3) respectivement du col de cygne (2) est une pince, un clip ou une agrafe en métal ou en matière plastique.

7. Dispositif lumineux mobile (1) selon la revendication 4, 5 ou 6, **caractérisé en ce que** le dispositif de couplage (5, 352 ; 35 ; 350) présente une partie de logement (35 ; 51) reliable au col de cygne (2), qui est de préférence fixée d'une seule pièce sur le coffret (30) du module d'énergie (3) respectivement est reliée à ce dernier ou au moyen d'un élément de fixation (52) avec le coffret (30) du module d'énergie (3).

8. Dispositif lumineux mobile (1) selon la revendication 7, **caractérisé**
a) **en ce que** l'élément de fixation (52) est accouplable à un élément de retenue (352) moulé de préférence d'une seule pièce sur le coffre (30) du module d'énergie (3); ou
b) **en ce que** l'élément de fixation (52) forme un profilé en U (521, 522, 523) servant au logement du module d'énergie (3) dont les parois latérales (521, 523) sont réalisées comme des agrafes qui maintiennent le module d'énergie (3); ou
c) **en ce que** l'élément de fixation métallique (52) est maintenu au moyen d'un aimant de couplage (350) de préférence dans une cavité (37) prévue dans le coffret (30) du module d'énergie (3); ou
d) **en ce que** l'élément de fixation (52) présente un aimant de montage (58) qui maintient le module d'énergie (3) muni d'un contre-élément magnétique dur ou tendre (830) à l'intérieur de l'élément de fixation (52).

9. Dispositif lumineux mobile (1) selon la revendication 8, **caractérisé en ce que** l'aimant (350) est de forme annulaire et est disposé dans le prolongement de la fenêtre de coffret (36) de sorte que la lumière dégagée par au moins une diode lumineuse (82) peut traverser l'aimant (350) et/ou la diode lumineuse (82) s'engage axialement dans les aimants (350).

10. Dispositif lumineux mobile (1) selon l'une des revendications 1 à 9, **caractérisé en ce que** le coffret 30 du module d'énergie (3) présente une zone élastique qui permet l'actionnement manuel de l'interrupteur (83).

11. Dispositif lumineux mobile (1) selon l'une des revendications 1 à 10, **caractérisé en ce que** le coffret (30) du module d'énergie (3) présente un point de rupture théorique (39) qui est réalisé de sorte que sa séparation ouvre au moins l'accès à l'unité d'alimentation de courant (81), d'une pile ou d'un accumulateur.

12. Dispositif lumineux mobile (1) selon l'une des revendications 1 à 10, **caractérisé en ce que** l'unité d'alimentation électrique (81) est reliée de préférence par une diode (85) à une bobine (86), qui est accouplable à une bobine (96) d'un chargeur (9), de préférence sans contact.

13. Dispositif lumineux mobile (1) selon l'une des revendications 1 à 12, **caractérisé en ce que** l'interrupteur (83) est réalisé et positionné et/ou coopère avec un contre-élément (830) logé mobile de sorte qu'il peut être fermé par action de l'aimant magnétique (58) ou de l'aimant d'accouplage (350).

14. Dispositif lumineux mobile (1) selon l'une des revendications 1 à 13, **caractérisé en ce qu'**au moins un conducteur d'ondes lumineuses (21) éventuellement un faisceau de conducteurs d'ondes lumineuses (21) sont flexibles et stables ou sont maintenus dans un corps tubulaire flexible et stable.

15. Dispositif lumineux mobile (1) selon l'une des revendications 1 à 14, **caractérisé en ce que** le coffret (30) du module d'énergie (3) présente une surface lisse.
